(19) Europäisches Patentamt
European Patent Office
Office européen des brevets

(11) EP 3 851 543 A1

(12) **EUROPEAN PATENT APPLICATION**
published in accordance with Art. 153(4) EPC

(43) Date of publication:
**21.07.2021 Bulletin 2021/29**

(21) Application number: **19860288.0**

(22) Date of filing: **11.09.2019**

(51) Int Cl.:
*C12Q 1/6869 (2018.01)*
*A61P 15/00 (2006.01)*
*A61P 35/00 (2006.01)*
*G01N 33/50 (2006.01)*
*G01N 33/53 (2006.01)*
*G01N 33/574 (2006.01)*
*A61K 45/00 (2006.01)*
*C12N 15/12 (2006.01)*

(86) International application number:
**PCT/JP2019/035774**

(87) International publication number:
**WO 2020/054782 (19.03.2020 Gazette 2020/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **13.09.2018 JP 2018171850**

(71) Applicants:
- **National Cancer Center**
  **Tokyo 104-0045 (JP)**
- **H.U. Group Research Institute G.K.**
  **Tokyo 192-0031 (JP)**

(72) Inventors:
- **ISHIHARA, Hiroki**
  **Tokyo 104-0045 (JP)**
- **USHIJIMA, Toshikazu**
  **Tokyo 104-0045 (JP)**

(74) Representative: **Hoffmann Eitle**
**Patent- und Rechtsanwälte PartmbB**
**Arabellastraße 30**
**81925 München (DE)**

(54) **METHOD FOR ESTIMATING BREAST CANCER CELL ABUNDANCE**

(57) The present invention provides an indicator with improved accuracy which is for predicting an effect of a medicinal therapy on breast cancer. Specifically, the present invention provides a method for estimating a breast cancer cell existence ratio and other methods, the method including:
(1) analyzing a methylation level of
(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues, in a sample derived from a human subject; and

(2) estimating the breast cancer cell existence ratio in the sample derived from the human subject, based on the methylation level analyzed at (1).

FIG. 1

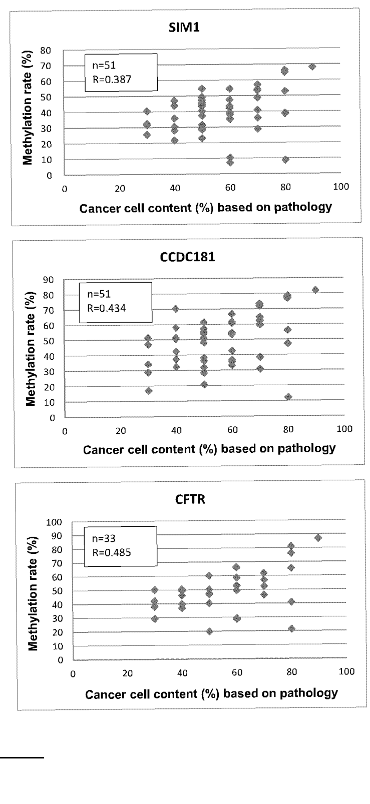


EP 3 851 543 A1

SEQUENCE LISTING

SEQUENCE LISTING

<110> NATIONAL CANCER CENTER
MIRACA RESEARCH INSTITUTE G.K.

<120> Method of estimating breast cancer cell existence ratio

<130> PA-R011233

<150> JP2018-171850
<151> 2018-09-13

<160> 12

<170> PatentIn version 3.5

<210> 1
<211> 2001
<212> DNA
<213> Homo sapiens

<400> 1
```
aacaactccc tccaaacgca cctctaggag ccgaatctgg tcaccggcgt accaaaacca      60

agccgggagt cgagtctgta gggggtgatt cgtgaggaca acaattgttt ttgggacaat     120

ttgacttttt cttgatgcta cctgttgtgt tttctcagat gagagaaaga gaaggcggtt     180

caaattagag ggtctctaaa agataattag tcagcaagag tcctggtgag cgtttgttta     240

aacagctcct attagggaac cgcgagcatc gttaagttca ttcatgtaga ctgtatcctt     300

tctgtaagga ataatcatgt tggacttttg tttcctgaaa caaaacccga caaagagttg     360

gctaatgttt aagataaagg actgttggga gctatgtatc tctatatcaa tcattctatt     420

ttgccctgaa atgtaagctt ttcccctagt gtccccctag ctgctcccca taggaaaact     480

ccccggccga gtttccacgg tgaaaaaaat cagccccaaa cccaagccat ttcgaaaaag     540

acgcacttct ttgggattaa tttaaagaca gttattgtat ttatttcctc tggcaatgaa     600

ttccgtgtcg ccatggtttc agagaaacga gaaagagaaa ataactcccc aaaccggcct     660

cgcgcacgcc gtcggccagc gactgccagc gggatgggcc tttgggcgaa ccctgcctcc     720

tccgccggta gacctaaccc gcaggcccga cctccgctgc ctccggacgt tcccggttct     780

gctgggccgc cgcgctggac tgagcgctca accacccgac cccagggagc tcgaggagcg     840

cgggtcgcgc acgacgtcgg gaaggcccag gaggccgcga gcagcggcgg caaccggaca     900

gcgccgaccc ggcaccctga gctcattagg agtccagcgg tcccgcgggt agtaagattc     960

agagcccgga gcctgggcgc gcggagggcg gctcggggcc gactcgtgtc actcagccct    1020

attggccaat gagcgcctcg cccctggccg cgccaggcca atgggaggcg agggggcttg    1080

tgagtggcat tgagggaggg cgggagagag gcggccccgg agtgaagttg aagctaaacc    1140

cttaagctat aaagaagtta cggggggcag ttttcggctt ccaattagga ataatagtga    1200

actggcttcg tagcaactac ggaggaccag gattctaaaa tcacctcact cgtcccaaag    1260
```

cttgcatcct cctctttctg ccacgcaccc ctcctccaat tcatgatcca gaaaagggag 1320

ccgggaatgc tctgctcctc tctgccggtg ggaagcggag aggccggcgg tgtcgctggg 1380

ttggacggta ggcatgagaa cagttaagag atgggcgccc ccgaaacctc tgccgcttgt 1440

ggggactgaa ggtaggtgaa gcagaagacg ccccgcgccc gcccagcagc cccgcagctc 1500

cgcggtggtg tgggagaggc cgcggcgcct cccacccccg ggggagcctg cgaggggctg 1560

tcgcgagcgc gccacctgtt aacctggcgc tgctgggccc cgcttgttgc agcccctgct 1620

gggcagccag agcgctgggt cgccttggga gtcccgagag acttggtgtg taagtgtcac 1680

tttttgagga atccctcaga cttgagaccc aagttaaaga ggtagccaag gtccagatct 1740

gccaaggtag ggagctgaaa ggcccctgcc tcgccttgga ggaattctga tttgcggaaa 1800

cctgctttcc tgggacgtgc gcccggcctg ggcgcggact cggggatccg cggcggaaga 1860

tcccttgcgg gtcttcagaa atatgtattt tgttgtttgg gcaacttccc aggggtctgg 1920

aggatgcggc tggcatgggt gagggaatgc cagcattatt tgcaggggc tgtctcgctg 1980

ccgatttacc cgtttcttta a 2001

<210> 2
<211> 2001
<212> DNA
<213> Homo sapiens

<400> 2
catattgggt acagtgtaca ctgctcaggt gatgggtgca ccaaaatctc agaaatcacc 60

gctaaagaac ttatccatgt aaccaaacac cacctgttcc cccaaaacct gatacttaaa 120

aaaaaagctg ctatcaccca ttatttgaaa aatacaaatt taggaggatg gtcagaggaa 180

aaaactatta acctaggagt tctgaaacct agggtctact tccagctttg gtaagtaact 240

ctacaactct acatccatta aaggagattt gactaaaatt ccttcaacct ttagcagtga 300

ataacctttt ttgggagcgt ctttttttcaa gcgctgaagc agtagcaaca ttaggtcatt 360

acaactacac cacatctagg aagtgcactc aatattacaa ttcattttaa ccttccattt 420

taaaagacct taagtctata acttgaagaa tatctaaaaa tttaatttta cattgcctct 480

cttggagaaa aggtgctctc catcacatca agtgatagaa atcctaataa taaacaattt 540

aaaaataatc cgaacacact ggattgtact gaggaaatat ttttacttct cccagttctt 600

atttatttct ggctgggaat ggatagttgt taaaatcatc tcaactttca ccacaatcta 660

aattcatctt gctttgcctt tatacataaa gataagcaga atttgagaaa attctagttg 720

accattttga catgcctcaa agctagagca aaaatcctat tttctttggc aactctcttg 780

ggtaaagttt tggttttgat taggaaaaaa agaagctgag ggtcaaaaat atggtaactc 840

attagcaatt tgcttatcta ctaatgaaaa ctaaaaacta cttttctacg ttaactgtga 900

agcacataac atatagaaat atagttttag aagttgtcct aagaaattct aaatctgacg 960

agagacagct acaagaggga aatttcacaa ccaacacagc ggcacttcgc gagtctctat 1020

aacgttagtg atgcgtggat tccttagtcg tccccttaga aacctcagct tcactggccc 1080

ttcgcaagca ccgggatggg tgccgggagg ggtcggtggt ctgaggatat tgcatgcgcc 1140

tgcgcagatt tttgtcgctc tcgtagttag agaggcccgg atggaggacg cagaggcacg 1200

ctgttgccat ggcagtgtgg tcctggctgc cgcggaggca ggtgccgggg tctcctttgc 1260

ctcaatgtga agagcttaga aagaggagga gaggagaact cccccggcca tctctgtgat 1320

cccagccgcc gcattttaca caggcaagag ggatatagag ggagagaagg gaaggacgat 1380

gcggagatgt taatggtggg atagagggta aaggtataaa cgtctggtat agctgggagg 1440

gagggtaggt agtgagggaa gaagctttct ggtggcgcct tatcctctgg agtccgaggt 1500

ccaccgagtc tggcactggt ggggaagtct ggtaacacct ttcttcacta tgtatgaagg 1560

atactcgtct gagagtagct tagactaaat ttttgtcata ttctttcccc tctcaaagtt 1620

cttgaacgtg gaaagtaata attttgtccc tgggagaaaa agtcactccc gaaattgtag 1680

cttccatgag gaggcaatgg tattcatttc gttggtggta ccttgggatc ctgaatacag 1740

attaaaatta tcacctaaag tccataaaat atttatcatc atactttgtg acagtttatg 1800

atttttaaa aacttactta taggatcaca gtcttggcag gatacacagg aataatgggc 1860

cagatggttt catgatgtat ggattagttt ttgcttaagt acttacttta tattaaaagg 1920

cattctcttt gacatgcttt cagttcttag atttttaagc aagcatatgt ttaaaacgct 1980

gtaagatggg acccaattat t 2001

<210> 3
<211> 2001
<212> DNA
<213> Homo sapiens

<400> 3
cggaagggca aagcagagct atgaaaacct gctgaacaca ttctttattt tcaacacagg 60

ttcttgtctt tccatcatga aatgcacatt ttatttgtac tgtatttggg tgaccacaag 120

tcaacaacaa gataattcac aagacccttg ccttagatgt gtcggcaata aagtaatcag 180

gccaaaattt ttactttcct ttgaattttt caattcaaac acaatgtatg cttgctttta 240

cacagtaggg ttcagggatt agagggttgg ctctttaaaa accgtcagag acacaggcaa 300

tcctacacaa aattctcaga aggaaggcgc ctacgcctgg gaatgcccag atgcccctca 360

gagagttgaa gatggcgttt ctctgagtca ggtcaaagtt aacacattac cttcgcttca 420

aagactgctt ggcttccttt cggtggatta gtcaagatgt tttgctgact gagactagga 480

aatctatagg agggcgggtt agtttacatt gttccttgtc attatcgcta aaacactcca 540

aagccttcct taaaaatgcg cactgggcta aaaaggatag acaaggaaca catcctgggc 600

cggtaattac gcaaagcatt atctcctctt acctccttgc agattttttt ttctctttca 660

gtacgtgtcc taagatttct gtgccaccct tggagttcac tcacctaaac ctgaaactaa 720

taaagcttgg ttcttttctc cgacacgcaa aggaagcgct aaggtaaatg catcagaccc 780

acactgccgc ggaacttttc ggctctctaa ggctgtattt tgatatacga aaggcacatt 840

ttccttccct tttcaaaatg caccttgcaa acgtaacagg aacccgacta ggatcatcgg 900

gaaaaggagg aggaggagga aggcaggctc cggggaagct ggtggcagcg ggtcctgggt 960

ctggcggacc ctgacgcgaa ggagggtcta ggaagctctc cggggagccg gttctcccgc 1020

cggtggcttc ttctgtcctc cagcgttgcc aactggacct aaagagaggc cgcgactgtc 1080

gcccacctgc gggatgggcc tggtgctggg cggtaaggac acggacctgg aaggagcgcg 1140

cgcgagggag ggaggctggg agtcagaatc gggaaaggga ggtgcggggc ggcgagggag 1200

cgaaggagga gaggaggaag gagcgggagg ggtgctggcg gggtgcgta gtgggtggag 1260

aaagccgcta gagcaaattt ggggccggac caggcagcac tcggctttta acctgggcag 1320

tgaaggcggg ggaaagagca aaaggaaggg gtggtgtgcg gagtaggggt gggtgggggg 1380

aattggaagc aaatgacatc acagcaggtc agagaaaaag ggttgagcgg caggcaccca 1440

gagtagtagg tctttggcat taggagcttg agcccagacg gccctagcag ggaccccagc 1500

gcccgagaga ccatgcagag gtcgcctctg gaaaaggcca gcgttgtctc caaacttttt 1560

ttcaggtgag aaggtggcca accgagcttc ggaaagacac gtgcccacga aagaggaggg 1620

cgtgtgtatg ggttgggttt ggggtaaagg aataagcagt ttttaaaaag atgcgctatc 1680

attcattgtt ttgaaagaaa atgtgggtat tgtagaataa aacagaaagc attaagaaga 1740

gatggaagaa tgaactgaag ctgattgaat agagagccac atctacttgc aactgaaaag 1800

ttagaatctc aagactcaag tacgctacta tgcacttgtt ttatttcatt tttctaagaa 1860

actaaaaata cttgttaata agtacctaag tatggtttat tggttttccc ccttcatgcc 1920

ttggacactt gattgtcttc ttggcacata caggtgccat gcctgcatat agtaagtgct 1980

cagaaaacat ttcttgactg a 2001

<210> 4
<211> 28
<212> DNA
<213> Artificial Sequence

<220>
<223> primer

<400> 4
ggtttagagg gtagtaagat ttagagtt 28

<210> 5
<211> 25
<212> DNA
<213> Artificial Sequence

<220>
<223> primer

<400> 5
aactaccccc cctaacttct ttata 25

<210> 6
<211> 20
<212> DNA
<213> Artificial Sequence

<220>
<223> primer

<400> 6
accaataaaa ctaaataaca 20

<210> 7
<211> 30
<212> DNA
<213> Artificial Sequence

<220>
<223> primer

<400> 7
gaagagagat agttataaga gggaaatttt 30

<210> 8
<211> 25
<212> DNA
<213> Artificial Sequence

<220>
<223> primer

<400> 8
accctctatc ccaccattaa catct 25

<210> 9
<211> 20
<212> DNA
<213> Artificial Sequence

<220>
<223> primer

<400> 9
gggaaatttt ataattaata 20

<210> 10
<211> 29

```
<212>  DNA
<213>  Artificial Sequence

<220>
<223>  primer

<400>  10
aaggaagagt taaggtaaat gtattagat                                 29


<210>  11
<211>  25
<212>  DNA
<213>  Artificial Sequence

<220>
<223>  primer

<400>  11
ccacctctct ttaaatccaa ttaac                                     25


<210>  12
<211>  18
<212>  DNA
<213>  Artificial Sequence

<220>
<223>  primer

<400>  12
ggagggttta ggaagttt                                             18
```

## Description

TECHNICAL FIELD

The present invention relates to a method for estimating a breast cancer cell existence ratio.

BACKGROUND ART

Evaluation of cancer cell fractions in a cancer tissue sample is important in order to prevent the accuracy of determination from decreasing due to unnecessary fractions (normal cells) mixed in, in a diagnosis of cancer by the use of a cancer marker (for example, determination of the risk of cancer development, determination of the development of cancer, prediction of an effect of a medicinal therapy on cancer (for example, Non Patent Literature 1) and a prognostic evaluation of a medicinal therapy on cancer).

Examples of a method for evaluating cancer cell fractions include (1) a pathological diagnosis, and (2) a method of, by a predetermined process, isolating cancer cells from a cancer tissue sample (including the cancer cells and non-cancer cells) collected by biopsy. However, there are problems that the method (1) requires the involvement of a skilled medical doctor and that the method (2) is complicated, for example.

To solve the above-mentioned problems, there has been proposed an evaluation of cancer cell fractions by analyzing a cancer tissue sample by the use of a marker ("fraction marker") specific to the cancer cell fractions. As such a fraction marker, for example, methylation markers for esophageal cancer, namely, TFAP2B, ARHGEF4, and RAPGEFL1 (Non Patent Literature 2), and methylation markers for gastric cancer, namely, OSR2 PPFIA3, and VAV3 (Non

Patent Literature 3) have been reported.

PRIOR ART REFERENCES

Non Patent Literature

Non Patent Literature 1: Fujii S, Yamashita S, Yamaguchi T, Takahashi M, Hozumi Y, Ushijima T, et al. Pathological complete response of HER2-positive breast cancer to trastuzumab and chemotherapy can be predicted by HSD17B4 methylation. Oncotarget.2017;8(12):19039-48.doi:10.18632/oncotarget.15118.
Non Patent Literature 2: Takahashi T, et al., PLoS one, 2013;8 (12) :e82302.
Non Patent Literature 3: Zong L, et al., Gastric cancer, 2016;19(2) :361-9.

SUMMARY OF INVENTION

PROBLEM TO BE SOLVED BY THE INVENTION

The object of the present invention is to provide a cancer marker for estimating the breast-cancer-cell content of breast cancer tissue. Another object of the present invention is to enhance the accuracy of an indicator configured to predict an effect of a medicinal therapy on breast cancer by determining the content of breast cancer cells.

SOLUTION TO PROBLEM

The inventors first conceived ideas of, in order to evaluate breast cancer cell fractions, searching a CpG site specific to breast cancer cells, the CpG site being methylated to a high degree in breast cancer cells, but methylated not at all or to a low degree in normal cells (non-breast cancer cells), and, in the above-mentioned search, selecting the CpG site not having a high incidence of copy number alterations of cells and being capable of targeting a broad range of patients. Then, the inventors comprehensively earnestly studied on more than 450,000 methylation candidate sites present in autosomes and sex chromosomes, and found specific CpG sites in SIM1 gene, CCDC181 gene, and CFTR gene, as such a CpG site. The inventors succeeded in, for example, utilizing methylation levels of these CpG sites as an indicator for a breast cancer cell existence ratio, and have completed the present invention.

That is, the present invention is as follows.

[1] A method for estimating a breast cancer cell existence ratio, the method comprising:

(1) analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues, in a sample derived from a human subject; and

(2) estimating the breast cancer cell existence ratio in the sample derived from the human subject, based on the methylation level analyzed at (1).

[2] The method according to [1], wherein the analyzing is carried out using one or two or more means selected from the group consisting of bisulfite, one or more primers, one or more nucleic acid probes, a restriction enzyme, an anti-methylated cytosine antibody, and a nanopore.
[3] The method according to [1] or [2], wherein the analyzing is carried out by a bisulfite sequencing method, a bisulfite pyrosequencing method, a methylation specific polymerase chain reaction (PCR) method, a restriction enzyme landmark genome scanning (RLGS) method, a single nucleotide primer extension (SNuPE) method, a CpG island microarray method, a MethyLight method, a COBRA method, a mass spectroscopy (mass array) method, use of a methylation specific restriction enzyme, a high resolution melting (HRM) analysis method, a nanopore analysis method, an ICON probe method, a methylation specific MLPA method, or an immunoassay.
[4] A method for predicting an effect of a medicinal therapy on breast cancer, the method comprising:

(1) measuring a value of a breast cancer marker in a sample derived from a human subject;
(2) analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues, in the sample derived from the human subject;

(3) correcting the value of the breast cancer marker measured at (1) by the methylation level analyzed at (2) to calculate a correction value of the breast cancer marker; and
(4) predicting the effect of the medicinal therapy on breast cancer, based on the correction value of the breast cancer marker corrected at (3).

[5] A method for determining breast cancer, the method comprising:

(1) analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues, in a sample derived from a human subject; and

(2) estimating a possibility of developing breast cancer, based on the methylation level analyzed at (1).

[6] A reagent for estimating a breast cancer cell existence ratio, the reagent comprising a means for analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues.

[7] A reagent for determining breast cancer, the reagent comprising a means for analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues.

[8] A kit, comprising:

(1) a means for analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues; and

(2) a means for measuring a breast cancer marker.

EFFECT OF THE INVENTION

The method for the estimation according to the present invention is useful, for example, for enhancing the accuracy of determination of breast cancer (for example, determination the risk of development of breast cancer, determination of the development of breast cancer, prediction of an effect of a medicinal therapy, and prognostic evaluation) by the use of a breast cancer marker.
The method for the prediction according to the present invention is useful, for example, as an indicator for determining the necessity of a surgical operation for a patient with breast cancer and determining the selection of a medicinal therapy on the patient.
The method for the determination according to the present invention is useful, for example, for making a diagnosis of breast cancer.
The reagent and the kit of the present invention are useful, for example, for simply performing the methods of the present invention.

BRIEF DESCRIPTION OF DRAWINGS

FIG. 1 is a diagram of graphs each illustrating a correlation between a cancer cell content evaluated by microscopic examination and a methylation level of SIM1, CCDC181, or CFTR.
FIG. 2 is a diagram of graphs each illustrating a correlation between the cancer cell content evaluated by microscopic examination and a methylation level (a higher methylation level to be selected) of a combination of two genes selected from SIM1, CCDC181, and CFTR.
FIG. 3 is a graph illustrating a correlation between the cancer cell content evaluated by microscopic examination and a methylation level (the highest methylation level to be selected) of a combination of three genes selected from SIM1, CCDC181, and CFTR.
FIG. 4 is a diagram of graphs illustrating values obtained by correcting measured values of methylation levels of HSD17B4 gene in a HER2-positive breast cancer tissue sample with a pathological complete response (pCR) or a non-pathological complete response (Non-pCR), by using the cancer cell content evaluated by microscopic examination and a methylation level of a combination of SIM1 and CCDC181.

EMBODIMENTS FOR CARRYING OUT THE INVENTION

(General Description)

A target cancer in the present invention is breast cancer. Breast cancer can be classified into subtypes, namely, HER2-positive breast cancer, Luminal A breast cancer, Luminal B breast cancer, and triple-negative breast cancer. The target cancer in the present invention may be any of the breast cancer subtypes, but, in a specific aspect, HER2-positive breast cancer is preferable.

In the present invention, "a breast cancer cell existence ratio" indicates the ratio of the number of breast cancer cells to the number of all the cells in a sample (that is, the total of the number of breast cancer cells and the number of non-breast cancer cells). That is, the breast cancer cell existence ratio is expressed by the following formula.

(breast cancer cell existence ratio) = (the number of breast cancer cells) / [(the number of breast cancer cells) + (the number of non-breast cancer cells)]

In the present invention, "fraction marker" denotes a marker specific to cancer cell fractions when a cell population, such as a sample derived from a human subject, is separated into the cancer cell fractions and non-cancer cell (normal cell) fractions. When the fraction marker is a gene, such a gene is sometimes referred to as "fraction marker gene".

Information on CpG sites of SIM1 gene, CCDC181 gene, and CFTR gene (breast cancer fraction marker genes) that are intended mainly to be analyzed in the present invention is as listed in Table 1 below (the position of a genome is based on human genome assembly hg38).

[Table 1]

Table 1. Breast Cancer Fraction Marker Gene

| No. | Gene | Chromosome | Nucleotide No. | Probe ID |
|---|---|---|---|---|
| 1 | SIM1 | 6 | 100465064 | cg27252696 |
| 2 | CCDC181 | 1 | 169427630 | cg24808280 |
| 3 | CFTR | 7 | 117479583 | cg09626894 |

The CpG site of SIM1 gene to be analyzed in the present invention can be defined as a CG portion consisting of nucleotide residues at positions 1,000 and 1,001 in the nucleotide sequence of SEQID NO:1 (nucleotides at positions 4 and 5 in a standard nucleotide sequence ggccgact present upstream of exon 1 of SIM1 gene) (when the ggc portion on the 5' side and the act portion on the 3' side are not mutated). The standard nucleotide sequence is intended to be referred to in order to identify a CpG site corresponding to a CpG site listed in Table 1 above (the same applies to CpG sites of other genes to be analyzed in the present invention). For a human subject having genomic DNA mutations, a person skilled in the art can suitably determine a CpG site corresponding to the CpG site present at the above-mentioned location by considering genomic DNA mutations. Hereinafter, "the CpG site of SIM1 gene" simply referred to in the present specification indicates the above-mentioned CpG site unless otherwise described.

The CpG site of CCDC181 gene to be analyzed in the present invention can be defined as a CG portion consisting of nucleotide residues at positions 1,000 and 1,001 in the nucleotide sequence of SEQ ID NO:2 (nucleotides at positions 4 and 5 in a standard nucleotide sequence cagcggca present in intron 2 of CCDC181 gene) (when the cag portion on the 5' side and the gca portion on the 3' side are not mutated). For a human subject having genomic DNA mutations, a person skilled in the art can suitably determine a CpG site corresponding to the CpG site present at the above-mentioned location by considering genomic DNA mutations. Hereinafter, "the CpG site of CCDC181 gene" simply referred to in the present specification indicates the above-mentioned CpG site unless otherwise described.

The CpG site of CFTR gene to be analyzed in the present invention can be defined as a CG portion consisting of nucleotide residues at positions 1,001 and 1,002 in the nucleotide sequence of SEQ ID NO:3 (nucleotides at positions 4 and 5 in a standard nucleotide sequence ctccgggg present upstream of exon 1 of CFTR gene) (when the ctc portion on the 5' side and the ggg portion on the 3' side are not mutated). For a human subject having genomic DNA mutations, a person skilled in the art can suitably determine a CpG site corresponding to the CpG site present at the above location by considering genomic DNA mutations. Hereinafter, "the CpG site of CFTR gene" simply referred to in the present specification indicates the above-mentioned CpG site unless otherwise described.

(Method for estimating a breast cancer cell existence ratio)

The present invention provides a method for estimating a breast cancer cell existence ratio.

The method for the estimation according to the present invention includes:

(1) analyzing a methylation level of

(a) a cytosine residue in the CpG site of SIM1 gene,
(b) a cytosine residue in the CpG site of CCDC181 gene,
(c) a cytosine residue in the CpG site of CFTR gene, or
(d) a combination of these cytosine residues, in a sample derived from a human subject; and

(2) estimating a breast cancer cell existence ratio in the sample derived from the human subject, based on the methylation level analyzed at (1).

At the step (1), as the sample, one or two or more samples selected from the group consisting of blood, body fluid, tissue, and a cell can be used, and a tissue sample corresponding to the above-mentioned cancer can be preferably used. In the present invention, in a genomic DNA contained in such a cancer tissue sample, a specific CpG site is analyzed. The genomic DNA can be suitably extracted from the cancer tissue sample by an arbitrary method. As the cancer tissue sample, a sample collected by biopsy, such as needle biopsy, can be used. The cancer tissue sample may be subjected to pretreatment. Examples of the pretreatment include extraction, cell fixation, tissue fixation, tissue slicing, cell dissociation treatment, heating, freezing, cold storage, and liquefaction. The cancer tissue sample is typically a sample of a cell population including cancer cells and normal cells (non-cancer cells).

The methylation to be analyzed in the present invention indicates methylation at position 5 in a cytosine residue.

The analysis of the methylation level can be performed by any method known in the art. For example, the analysis can be performed using one or two or more analytical means selected from the group consisting of bisulfite, a primer, a nucleic acid probe, a restriction enzyme, an anti-methylated cytosine antibody, and a nanopore.

Bisulfite converts non-methylated cytosine to uracil, whereas bisulfite does not convert methylated cytosine to uracil. Thus, due to such a nature, bisulfite is commonly used in combination of other means for analysis of methylated cytosine.

The primers indicate a primer for sequencing, a primer for gene amplification (for example, a polymerase-chain-reaction (PCR) primer), or a combination thereof.

The primer can be suitably designed so as to analyze a target CpG site. For example, the primer can be designed so as to anneal to a downstream region of the target CpG site (for SIM1 gene, any region in a portion consisting of nucleotide residues at positions 1,002 to 2,001 in the nucleotide sequence of SEQ ID NO:1; for CCDC181 gene, any region in a portion consisting of nucleotide residues at positions 1,002 to 2,001 in the nucleotide sequence of SEQ ID NO:2; or, for CFTR gene, any region in a portion consisting of nucleotide residues at positions 1,003 to 2,001 in the nucleotide sequence of SEQ ID NO:3), or, in order to amplify a gene region including the target CpG site, the primer can be designed so that a sense strand and an antisense strand are annealed in an upstream region of the target CpG site (for SIM1 gene, any region in a portion consisting of nucleotide residues at positions 1 to 999 in the nucleotide sequence of SEQ ID NO:1; for CCDC181 gene, any region in a portion consisting of nucleotide residues at positions 1 to 999 in the nucleotide sequence of SEQ ID NO:2; or, for CFTR gene, any region in a portion consisting of nucleotide residues at positions 1 to 1,000 in the nucleotide sequence of SEQ ID NO:1) and a downstream region of the target CpG site (any region mentioned as the downstream region above).

The nucleic acid probe can be designed so as to hybridize with a region including the target CpG site (for SIM1 gene, a portion including: a portion including the CG portion consisting of nucleotide residues at positions 1,000 to 1,001 in the nucleotide sequence of SEQ ID NO:1; for CCDC181 gene, a portion including the CG portion consisting of nucleotide residues at positions 1,000 to 1,001 in the nucleotide sequence of SEQ ID NO:2; or, for CFTR gene, a portion including the CG portion consisting of nucleotide residues at positions 1,001 to 1,002 in the nucleotide sequence of SEQ ID NO:3), or an upstream region thereof (any region mentioned as the upstream region above) or a downstream region thereof (any region mentioned as the downstream region above). The nucleic acid probe can be used in a free form or in a form immobilized to a solid phase. Examples of the solid phase include particles (for example, magnetic particles); supports, such as array, membrane (for example, nitrocellulose membrane and filter paper), and columns; and containers, such as plates (for example, multiwell plates) and tubes. Examples of a material for the solid phase include glasses, plastics, and metals. The nucleic acid probe may be the nucleic acid probe described in detail in the analysis of methylated cytosine using the anti-methylated cytosine antibody.

The restriction enzyme indicates a methylation-specific or -nonspecific restriction enzyme, and can be used suitably in combination with the above-mentioned bisulfite, the primer, or the nucleic acid probe.

Specific examples of an analytical method using the analytical means as described above include a DNA methylation sequencing method, a bisulfite sequencing method, a bisulfite pyrosequencing method, a methylation specific PCR method, a restriction enzyme landmark genome scanning (RLGS) method, a single nucleotide primer extension (SNuPE) method, a CpG island microarray method, a MethyLight method, a COBRA method, a mass spectroscopy (mass array) method, use of a methylation specific restriction enzyme, a high resolution melting (HRM) analysis method, a nanopore analysis method, an ICON probe method, and a methylation specific multiplex ligation-dependent probe amplification (MLPA) method. These methods are well-known in the art (for example, Japanese Patent Application Laid-open No. 2012-090555, Japanese Patent Application Laid-open No. 2014-036672, Japanese Unexamined Patent Application Publication (Translation of PCT Application) No. 2010-538638, and Republication of PCT International Publication No. 2009-136501).

Alternatively, the analysis can be performed using an anti-methylated cytosine antibody. The analysis of methylated cytosine by the use of the anti-methylated cytosine antibody is well-known in the art (for example, WO 2015/025862; WO 2015/025863; WO 2015/025864; WO 2016/052368; Japanese Patent Application Laid-open No. 2012-230019; DNA Research 13, 37-42 (2006); and Anal. Chem. 2012,84, 7533-7538). The anti-methylated cytosine antibody may be used in combination with one or two or more of the above-mentioned analytical means. Specific examples of a method using the above-mentioned analytical means include a method of using the anti-methylated cytosine antibody and a heterogeneous nucleic acid probe (for example, a normal RNA probe or a modified RNA probe) in combination (for example, WO 2015/025862); a method of using the anti-methylated cytosine antibody and a solid phase probe and a capture probe in combination (for example, WO 2015/025863); a method of using the anti-methylated cytosine antibody and an absorbent polynucleotide and a capture probe in combination (for example, WO 2015/025864); and a method of using the anti-methylated cytosine antibody and a modified nucleobase paired heterogeneous nucleic acid probe in combination (for example, WO 2016/052368). The nucleic acid probe to be used in combination with the anti-methylated cytosine antibody may be designed so that, when the nucleic acid probe is hybridized with a DNA strand including the above-mentioned CpG site subjected to be analyzed in the present invention to form a double strand structure consisting of the DNA strand and the nucleic acid probe, a methylcytosine residue in the CpG site forms a portion unpaired with the nucleic acid probe (in other words, not bound in a complementary manner) (for example, WO 2015/025862). Thus, the nucleic acid probe may have a nucleotide residue (for example, a cytosine residue, a thymine residue, an adenine residue, and an uracil residue), other than a guanine residue capable of binding complementarily to the methylcytosine residue as a nucleotide residue corresponding to the unpaired portion. Alternatively, the nucleic acid probe may be designed so as not to form such unpaired portion (WO 2016/052368).

The method of using the anti-methylated cytosine antibody can be performed by any immunological method known in the art. Specific examples of such method include an enzyme immunoassay (EIA) (for example, chemiluminescent enzyme immunoassay (CLEIA), enzyme-linked immune-sorbent assay (ELISA)), a fluorescent immunoassay, a chemiluminescent immunoassay, an electrochemical luminescent immunoassay, an agglutination method, immunostaining, a flow cytometry method, a biolayer interferometry method, an In Situ PLA method, a chemical amplification type luminescence proximity homogeneous assay, a line blot method, and a western blot method.

The methylation level to be analyzed in the present invention is the ratio of methylation of cytosine residues in the above-mentioned CpG in cancer cells. Measurement of the methylation level is well-known in the art (for example, Japanese Patent Application Laid-open No. 2012-090555, Japanese Patent Application Laid-open No. 2014-036672, Japanese Unexamined Patent Application Publication (Translation of PCT Application) No. 2010-538638, and Republication of PCT International Publication No. 2009/136501.

The methylation level to be analyzed in the present invention may be a combination of methylation levels of cytosine residues in the CpG sites of SIM1 gene, CCDC181 gene, and CFTR gene. The combination of methylation levels as described as the above-mentioned (d) is a combination of two or more (that is, two or three) of these methylation levels, and specifically a combination of methylation levels of cytosine residues in the CpG sites of a combination of SIM1 gene and CCDC181 gene, a combination of SIM1 gene and CFTR gene, a combination of CCDC181 gene and CFTR gene, or a combination of SIM1 gene, CCDC181 gene, and CFTR gene. As the methylation level in the combination, for example, any of the highest methylation level in the combination, the lowest methylation level in the combination, and the average methylation level in the combination may be adopted, and the highest methylation level is preferably adopted.

The breast cancer cell existence ratio in the sample derived from the human subject can be estimated, based on the analyzed methylation level at the step (2).

(Method for predicting effect of medicinal therapy on breast cancer)

The present invention also provides a method for predicting an effect of a medicinal therapy on breast cancer.

Examples of the medicinal therapy on breast cancer include a treatment with an anticancer agent. Examples of the anticancer agent include microtubule inhibitors, anticancer antibiotics, topoisomerase inhibitors, platinum formulations, and alkylating agents. The anticancer agent may also be a molecular targeted drug. Examples of the molecular targeted drug include HER2 inhibitors, EGFR inhibitors (for example, gefitinib, lapatinib, erlotinib, and cetuximab), c-MET inhibitors (for example, PHA-665752, SU11274, and XL-880), ALK inhibitors (for example, WHI-P154, TAE684, and PF-2341066), PDGFR inhibitors (for example, imatinib, desatinib, and valatinib), and c-KIT inhibitors (for example, sunitinib, masitinib, and motesanib).

The medicinal therapy on breast cancer can be suitably selected in accordance with a subtype of breast cancer.

Examples of the medicinal therapy on HER2-positive breast cancer include a treatment with an HER2 inhibitor. HER2 is receptor type tyrosine kinase belonging to the same family as epithelial growth factor (EGFR). HER2 has a structure in which an extracellular domain, a transmembrane domain, and an intracellular domain having tyrosine kinase activity are linked on a single strand (see, for example, Coussens, et al., Science, 1985, Vol. 230, No. 4730, pp.1132-1139, and Genebank Accession No. NP_001005862.1). Examples of the HER2 inhibitor include antibodies (for example, trastuzumab and pertuzumab), low molecular organic compounds (for example, HER2 tyrosine kinase inhibitors, such as lapatinib, neratinib, and afatinib), and drug-conjugated antibodies (for example, T-DM1) .

Examples of the medicinal therapy on Luminal A breast cancer include hormonotherapy and chemotherapy. Examples of the hormonotherapy include therapies using luteinizing hormone-releasing hormone (LH-RH) agonist formulations, anti-estrogen drugs, progesterone drugs, and aromatase inhibitors. Examples of the chemotherapy include therapies using topoisomerase inhibitors, microtubule agents, alkylating agents, and antimetabolites.

Examples of the medicinal therapy on Luminal B breast cancer include hormonotherapy and chemotherapy. Examples of the hormonotherapy include therapies using LH-RH agonist formulations, anti-estrogen drugs, progesterone drugs, and aromatase inhibitors. Examples of the chemotherapy include therapies using topoisomerase inhibitors, microtubule agents, alkylating agents, and antimetabolites.

Examples of the medicinal therapy on triple-negative breast cancer include chemotherapy. Examples of the chemotherapy include therapies using topoisomerase inhibitors, microtubule agents, alkylating agents, and antimetabolites.

In the medicinal therapy on the cancer, a plurality of anticancer agents can be used in combination. Examples of the combination of a plurality of anticancer agents include a combination of two or more of the above-mentioned anticancer agents and a combination of one or more of the above-mentioned anticancer agents and other anticancer agents. Examples of the other anticancer agents include microtubule inhibitors such as taxane-based anticancer agents (for example, paclitaxel), anthracycline-based anticancer agents (for example, ADRIACIN), and aromatase inhibitors (for example, letrozole).

The method for the prediction according to the present invention includes:

(1) measuring a value of a breast cancer marker in a sample derived from a human subject;
(2) analyzing a methylation level of

  (a) a cytosine residue in the CpG site of SIM1 gene,
  (b) a cytosine residue in the CpG site of CCDC181 gene,
  (c) a cytosine residue in the CpG site of CFTR gene, or
  (d) a combination of these cytosine residues, in the sample derived from the human subject;

(3) correcting the value of the breast cancer marker measured at (1) by the methylation level analyzed at (2) to calculate a correction value of the breast cancer marker; and
(4) predicting an effect of a medicinal therapy on breast cancer, based on the correction value of the breast cancer marker corrected at (3).

The breast cancer marker at the step (1) is a marker serving as a breast cancer indicator, and examples thereof include a DNA methylation level (for example, cg15896301 of HSD17B4: Non Patent Literature 3).

The CpG sites of the fraction marker genes (SIM1 gene, CCDC181 gene, and CFTR gene) at the step (2) and a means for analyzing the methylation level are as described above.

The correction of the value of the breast cancer marker at the step (3) is such that, based on the value of the breast cancer marker measured in the sample derived from the human subject (the sample including breast cancer cells and non-breast cancer cells), a value of the breast cancer marker that can correspond to a breast cancer cell more purely is calculated. Thus, the correction value of the breast cancer marker can be calculated, for example, by the following formula.

Correction value of breast cancer marker = (value of breast cancer marker measured in sample derived from human subject) / (breast cancer cell existence ratio in sample derived from human subject)

In the method of predicting an effect of a medicinal therapy on breast cancer, based on a value of the breast cancer marker, the step (4) can be performed using the correction value of the breast cancer in place of the value of the breast cancer marker. With the use of the correction value of the breast cancer marker, an effect of the medicinal therapy is expected to be predicted with high accuracy.

(Method for determining breast cancer)

The present invention also provides a method for determining breast cancer.
The determination of breast cancer indicates the determination of whether the possibility that a human subject has been affected by breast cancer is high or low.
The method for the determination according to the present invention includes:

(1) analyzing a methylation level of

(a) a cytosine residue in the CpG site of SIM1 gene,
(b) a cytosine residue in the CpG site of CCDC181 gene,
(c) a cytosine residue in the CpG site of CFTR gene, or
(d) a combination of these cytosine residues, in a sample derived from a human subject; and

(2) estimating the possibility of developing breast cancer, based on the methylation level analyzed at (1).

The sample derived from the human subject and the CpG sites of the fraction marker genes (SIM1 gene, CCDC181 gene, and CFTR gene) and the means for analyzing the methylation level at the step (1) are as described above.
At the step (2), it can be determined that, when the methylation level is equal to or higher than a criteria value, there is a possibility that the human subject has been affected by breast cancer, and/or, when the methylation level is lower than the criteria value, the possibility that the human subject has been affected by breast cancer is low. As such a criteria value, a cutoff value suitably set so as to differentiate between breast cancer patients and healthy persons can be used. As the cutoff value of the methylation level, for example, any percentage value within a range of 10% to 30% can be adopted, but, any percentage value within a range of 15% to 25% is preferably adopted, and 20% is more preferably adopted.

(Reagent and Kit)

The present invention also provides a reagent for estimating a breast cancer cell existence ratio.
The reagent for the estimation according to the present invention includes a means for analyzing the methylation level of:

(a) a cytosine residue in the CpG site of SIM1 gene;
(b) a cytosine residue in the CpG site of CCDC181 gene;
(c) a cytosine residue in the CpG site of CFTR gene; or
(d) a combination of these cytosine residues.

The CpG sites of the fraction marker genes (SIM1 gene, CCDC181 gene, and CFTR gene) and the means for analyzing the methylation level in the reagent for the estimation according to the present invention are as described above.
The reagent for the estimation according to the present invention can be used to analyze the methylation level of the fraction marker gene in a sample derived from a human subject by the use of the means for analyzing the methylation level, and estimate a breast cancer cell existence ratio in the sample derived from the human subject, based on the analyzed methylation level.
The present invention also provides a reagent for determining breast cancer.
The reagent for the determination according to the present invention includes a means for analyzing the methylation level of:

(a) a cytosine residue in the CpG site of SIM1 gene;
(b) a cytosine residue in the CpG site of CCDC181 gene;
(c) a cytosine residue in the CpG site of CFTR gene; or
(d) a combination of these cytosine residues.

The CpG sites of the fraction marker genes (SIM1 gene, CCDC181 gene, and CFTR gene) and the means for analyzing the methylation level in the reagent for the determination according to the present invention are as described above.
The reagent for the determination according to the present invention can be used to analyze the methylation level of the fraction marker gene in a sample derived from a human subject by the use of the means for analyzing the methylation level, and determine breast cancer in the human subject, based on the analyzed methylation level.
The present invention also provides a kit.
The kit according to the present invention includes:

(1) a means for analyzing the methylation level of

(a) a cytosine residue in the CpG site of SIM1 gene,
(b) a cytosine residue in the CpG site of CCDC181 gene,
(c) a cytosine residue in the CpG site of CFTR gene, or
(d) a combination of these cytosine residues, and

(2) a means for measuring a breast cancer marker.

The CpG sites of the fraction marker genes (SIM1 gene, CCDC181 gene, and CFTR gene), the means for analyzing the methylation level, and the means for measuring the breast cancer marker in the reagent for determining breast cancer according to the present invention are as described above.
The kit according to the present invention has various uses, and, for example, can be used for predicting an effect of a medicinal therapy on breast cancer.

EXAMPLES

Hereinafter, the present invention will be described in detail by using examples, but the present invention is not limited to these examples.

1. Sample

1-1. Biological Sample

As samples derived from breast cancer patients, 195 breast cancer tissue sample specimens were obtained from 195 breast cancer patients. The breast cancer tissue sample of each of the patients was collected by a needle biopsy from the patient with a diagnosis of breast cancer. The sample was fixed using the PAXgene Tissue System (Qiagen, Hilden, Germany) and embedded in low-melting paraffin. From the sample, 10 slices of 10 μm sections were made and DNA was extracted therefrom. At the same time, the sample was subjected to microscopic examination to determine a cancer cell fraction. For 61 specimens derived from HER2-positive breast cancer patients out of the above-mentioned 195 specimens, their therapeutic responses to trastuzumab were determined, and a state in which there was no residual cancer cells in the sample was defined as pathological complete response (pCR), and a state other than the above-mentioned state was defined as non-pathological complete response (Non-pCR). Furthermore, 10 specimens out of the 61 specimens were separated into cancer cells and non-cancer cells by laser capture microdissection (LCM).

1-2. Cultured Cell Line Sample

As cultured breast cancer cell line samples, a total of 20 breast cancer cell lines (BT-474, SK-BR-3, MDA-MB-453, HCC38, MDA-MB-231, T-47D, Hs578T, MCF7, UACC-3199, ZR-75-1, BT-20, MDA-MB-436, HCC1937, MDA-MB-468, HCC1428, BT-549, AU565, HCC1395, MDA-MB-157, and HCC1954) were purchased from the American Type Culture Collection (Rockville, MD).
As a cultured normal cell line sample, Human Mammary Epithelial Cells (HMECs) were purchased from Cambrex (East Rutherford, NL).

2. Measurement of DNA Methylation Level of Fraction Marker

2-1. Method for Measuring DNA Methylation Level

A gene-specific DNA methylation level was analyzed by bisulfite pyrosequencing. Specifically, the bisulfite pyrosequencing was conducted using the following procedure. A genomic DNA was digested with BamHI, and 1 μg of the resultant BamHI-digested genomic DNA was used for bisulfite modification, as previously reported (Yamashita S, Takahashi S, McDonell N, Watanabe N, Niwa T, Hosoya K, et al. Methylation silencing of transforming growth factor-beta receptor type II in rat prostate cancers. Cancer research. 2008;68(7):2112-21). The bisulfite modified DNA was suspended in 40 μl of a TE buffer solution, and an aliquot of 1 μl was used for bisulfite pyrosequencing, as previously reported (Yoshida T, Yamashita S, Takamura-Enya T, Niwa T, Ando T, Enomoto S, et al. Alu and Satalpha hypomethylation in Helicobacter pylori-infected gastric mucosae. International journal of cancer. 2011;128(1):33-9). A target genomic region was amplified by a biotinylated primer. A PCR product labelled with biotin was annealed to a 0.2 μM pyrosequencing primer, and pyrosequencing was carried out using the PSQ 96 Pyrosequencing System (QIAGEN, Valencia, CA, USA). A methylation level was obtained using the PSQ Assay Design software (QIAGEN).

2-2. Analysis of Methylation Level of Fraction Marker

2-2-1. Screening of Fraction Marker

From 477,344 CpG sites in autosomes and sex chromosomes, CpG sites having a low methylation level in the cultured normal cell lines (non-cancer cells) and having a high methylation level in the cultured breast cancer cell lines prepared at the step 1-2 were searched, and as a result, 3 CpG sites were found out as fraction markers. Table 1 lists details of the CpG sites.

2-2-2. Correlation between Breast Cancer Estimation Using Fraction Marker and Cancer Cell Content Based on Pathology

To determine the accuracy of breast cancer estimation using the fraction markers, a correlation between a cancer cell fraction estimated by each of the fraction markers (SIM1, CCDC181, and CFTR) or a combination thereof and the cancer cell content estimated by microscopic examination was determined in 51 HER2-positive breast cancer sample specimens (as for CFTR, 33 specimens). In the case where the fraction markers were used in combination, the highest one of the methylation levels of the three markers was adopted. FIGS. 1 to 3 illustrate the results. A significant correlation between the cancer cell fraction estimated by the fraction marker and the cancer cell content based on pathology was confirmed ($R = 0.38$ to $0.77$). Hence, these fraction markers were considered as markers capable of estimating a breast cancer cell fraction.

3. Correction of HSD17B4 Methylation Level by Breast Cancer Cell Fraction

3-1. Measurement of Value of Breast Cancer Marker

For 61 HER2-positive breast cancer sample specimens having been subjected to the determination of pCR and Non-pCR at the above-described step 1-1, the methylation level of HSD17B4 gene was measured. The methylation level of HSD17B4 was measured by bisulfite pyrosequencing in accordance with the above-described step 2-1 (see Non Patent Literature 3).

3-2. Correction of Value of Breast Cancer Marker

The methylation level of HSD17B4 measured at the above-described step 3-1 was corrected by the fraction marker or by a breast cancer cell fraction estimated by microscopic examination, as follows: [corrected HSD17B4 methylation level = 100 × (HSD17B4 methylation level)/ (cancer cell fraction)].

3-3. Statistical Analysis

Correlation analyses were performed using the Pearson's product-moment correlation coefficients. Differences in corrected methylation level of HSD17B4 between trastuzumab responders (pCR) and non-responders (non-pCR) were evaluated by the Mann-Whitney U test. All the analyses were performed using PASW statistics version 18.0 (SPSS Japan Inc., Tokyo, Japan), and two-sided p-values < 0.05 were considered statistically significant.

3-4. Application of Cancer Cell Fraction Marker to Correction of Methylation Level of HSD17B4

By estimating a cancer cell fraction, the extent to which a cancer cell could correct the methylation level of HSD17B4 was evaluated. Based upon raw data on methylation, no significant difference of the methylation level of HSD17B4 was observed between pCR and non-pCR samples (p = 0.245) (FIG. 4). In contrast, after the correction, the methylation level was significantly higher in the pCR specimens than in the non-pCR specimens (microscopic examination: p = 0.0001; fraction marker: p = 0.0004). Sensitivity and specificity to predict pCR (Table 2) before the correction were 13.6 % and 94.9 %, respectively. The sensitivity and specificity after the correction by the DNA methylation marker (59.1 % and 84.6 %) were equivalent to those corrected by microscopic examination (59.1% and 87.2%). In Table 2, the methylation levels of HSD17B4 are classified into high level and low level by using a cutoff value (50%) based on the previous report (the above-mentioned Fujii S et al). Here, pCR denotes pathological complete response.

[Table 2]

Table 2. Predictive performance of HSD17B4 methylation before correction, after correction by microscopic examination, and after correction by methylation fraction marker

| | HSD17B4 methylat ion | Number of pCR samples | Number of Non-pCR samples | Sensit ivity (%) | Specif icity (%) | Positive predicti ve value (%) |
|---|---|---|---|---|---|---|
| Before correction | high | 3 | 2 | 13.6 | 94.9 | 60.0 |
| | low | 19 | 37 | | | |
| Correction by microscopic examination | high | 13 | 5 | 59.1 | 87.2 | 72.2 |
| | low | 9 | 34 | | | |
| Correction by methylation fraction marker | high | 13 | 6 | 59.1 | 84.6 | 68.4 |
| | low | 9 | 33 | | | |

4. Evaluation of Applicability to Subtypes of Breast Cancer

For 134 breast cancer sample specimens (HER2-positive breast cancer, Luminal A breast cancer, Luminal B breast cancer, and triple-negative breast cancer), other than 61 specimens having been subjected to the pCR/Non-pCR determination at the 1-1, the methylation levels of fraction markers (SIM1, CCDC181, and CFTR) were measured in a state in which non-cancer cells were mixed in as well. With a methylation level of 20% being taken as cutoff, a specimen for which any of the three markers indicated a methylation level equal to or higher than the cutoff was determined to be "positive". Table 3 lists the results.

In Table 3, HER2 denotes HER2-positive breast cancer, and TNBC denotes triple-negative breast cancer.

[Table 3-1]

Table 3. Methylation levels of SIM1, CCDC181, and CFTR in subtype breast cancer samples

| Sample No. | Fraction marker | | | Subtype |
|---|---|---|---|---|
| | CFTR | CCDC181 | SIM1 | |
| 1 | 42.07 | 37.94 | 29.58 | HER2 |
| 2 | 33.06 | 31.54 | 6.88 | HER2 |
| 3 | 19.19 | 34.19 | 14.68 | HER2 |
| 4 | 18.66 | 15.78 | 9.37 | HER2 |
| 5 | 68.70 | 67.19 | 45.41 | HER2 |
| 6 | 51.59 | 55.62 | 48.43 | HER2 |
| 7 | 3.91 | 13.63 | 1.30 | HER2 |

(continued)

| Table 3. Methylation levels of SIM1, CCDC181, and CFTR in subtype breast cancer samples | | | | |
|---|---|---|---|---|
| Sample No. | Fraction marker | | | Subtype |
| | CFTR | CCDC181 | SIM1 | |
| 8 | 55.12 | 63.72 | 42.88 | HER2 |
| 9 | 40.44 | 50.37 | 21.37 | HER2 |
| 10 | 28.99 | 59.85 | 28.19 | HER2 |
| 11 | 57.59 | 62.77 | 52.91 | HER2 |
| 12 | 45.95 | 47.05 | 36.35 | HER2 |
| 13 | 69.39 | 40.21 | 51.05 | HER2 |
| 14 | 24.48 | 19.51 | 11.81 | HER2 |
| 15 | 58.43 | 57.76 | 8.10 | HER2 |
| 16 | 23.97 | 25.65 | 10.92 | HER2 |
| 17 | 26.98 | 35.26 | 10.21 | Luminal A |
| 18 | 69.25 | 57.52 | 29.72 | Luminal A |
| 19 | 61.31 | 48.92 | 11.68 | Luminal A |
| 20 | 48.41 | 58.61 | 6.99 | Luminal A |
| 21 | 6.19 | 56.46 | 22.51 | Luminal A |
| 22 | 77.68 | 58.61 | 6.25 | Luminal A |
| 23 | 4.82 | 19.86 | 15.69 | Luminal A |
| 24 | 82.68 | 80.51 | 74.21 | Luminal A |
| 25 | 51.97 | 63.21 | 37.92 | Luminal A |
| 26 | 51.34 | 31.61 | 8.04 | Luminal A |
| 27 | 52.10 | 64.00 | 55.91 | Luminal A |
| 28 | 39.44 | 30.71 | 18.08 | Luminal A |
| 29 | 49.89 | 45.83 | 25.38 | Luminal A |
| 30 | 72.36 | 72.27 | 55.46 | Luminal A |
| 31 | 15.27 | 52.53 | 11.16 | Luminal A |
| 32 | 45.02 | 63.33 | 30.80 | Luminal A |
| 33 | 33.63 | 34.80 | 27.24 | Luminal A |
| 34 | 61.08 | 66.59 | 54.48 | Luminal A |
| 35 | 3.96 | 61.97 | 41.23 | Luminal A |
| 36 | 71.90 | 50.45 | 28.11 | Luminal A |
| 37 | 34.39 | 12.50 | 19.67 | Luminal A |
| 38 | 21.05 | 13.45 | 29.56 | Luminal A |
| 39 | 3.48 | 10.76 | 7.37 | Luminal A |
| 40 | 60.93 | 67.48 | 32.83 | Luminal A |

[Table 3-2]

| Sample No. | Fraction marker | | | Subtype |
|---|---|---|---|---|
| | CFTR | CCDC181 | SIM1 | |
| 41 | 31.98 | 14.53 | 8.73 | Luminal A |
| 42 | 30.03 | 41.18 | 33.43 | Luminal A |
| 43 | 33.90 | 40.26 | 21.07 | Luminal A |
| 44 | 42.82 | 63.38 | 28.16 | Luminal A |
| 45 | 44.60 | 44.70 | 33.34 | Luminal A |
| 46 | 18.53 | 31.90 | 11.72 | Luminal A |
| 47 | 25.16 | 50.38 | 15.76 | Luminal A |
| 48 | 39.96 | 50.47 | 11.26 | Luminal A |
| 49 | 70.60 | 61.07 | 39.86 | Luminal A |
| 50 | 13.30 | 48.98 | 2.52 | Luminal A |
| 51 | 38.84 | 49.35 | 11.99 | Luminal A |
| 52 | 48.58 | 55.38 | 11.72 | Luminal A |
| 53 | 46.48 | 61.26 | 27.55 | Luminal A |
| 54 | 19.18 | 24.87 | 9.03 | Luminal A |
| 55 | 24.79 | 25.84 | 54.06 | Luminal A |
| 56 | 74.21 | 75.66 | 11.53 | Luminal A |
| 57 | 8.97 | 13.89 | 6.33 | Luminal A |
| 58 | 32.10 | 44.26 | 15.72 | Luminal A |
| 59 | 50.81 | 63.22 | 32.64 | Luminal A |
| 60 | 24.53 | 41.47 | 14.48 | Luminal A |
| 61 | 37.95 | 36.71 | 11.08 | Luminal A |
| 62 | 73.98 | 62.83 | 45.52 | Luminal A |
| 63 | 33.80 | 24.44 | 23.29 | Luminal A |
| 64 | 14.66 | 18.98 | 14.20 | Luminal A |
| 65 | 78.07 | 72.58 | 65.08 | Luminal A |
| 66 | 4.24 | 85.28 | 73.66 | Luminal A |
| 67 | 15.95 | 55.32 | 18.63 | Luminal A |
| 68 | 6.89 | 14.51 | 1.85 | Luminal A |
| 69 | 48.85 | 13.42 | 21.29 | Luminal B |
| 70 | 35.46 | 54.00 | 35.37 | Luminal B |
| 71 | 5.02 | 11.67 | 3.70 | Luminal B |
| 72 | 46.05 | 70.31 | 46.25 | Luminal B |
| 73 | 31.45 | 55.38 | 24.09 | Luminal B |
| 74 | 46.48 | 48.81 | 4.51 | Luminal B |
| 75 | 39.63 | 43.43 | 27.63 | Luminal B |
| 76 | 57.40 | 13.48 | 39.74 | Luminal B |
| 77 | 73.11 | 11.49 | 1.69 | Luminal B |

(continued)

| Sample No. | Fraction marker | | | Subtype |
|---|---|---|---|---|
| | CFTR | CCDC181 | SIM1 | |
| 78 | 48.86 | 38.63 | 38.94 | Luminal B |
| 79 | 17.87 | 10.96 | 38.35 | Luminal B |
| 80 | 23.83 | 22.61 | 5.51 | Luminal B |

[Table 3-3]

| Sample No. | Fraction marker | | | Subtype |
|---|---|---|---|---|
| | CFTR | CCDC181 | SIM1 | |
| 81 | 29.43 | 43.67 | 17.69 | Luminal B |
| 82 | 45.53 | 56.19 | 44.25 | Luminal B |
| 83 | 59.17 | 49.79 | 38.90 | Luminal B |
| 84 | 31.03 | 39.80 | 15.88 | Luminal B |
| 85 | 22.18 | 21.40 | 35.22 | Luminal B |
| 86 | 7.65 | 12.45 | 10.57 | Luminal B |
| 87 | 47.71 | 24.02 | 6.55 | Luminal B |
| 88 | 57.13 | 59.70 | 45.00 | Luminal B |
| 89 | 30.09 | 27.03 | 17.36 | Luminal B |
| 90 | 72.99 | 58.14 | 47.54 | Luminal B |
| 91 | 50.89 | 55.05 | 35.10 | Luminal B |
| 92 | 86.06 | 86.34 | 77.75 | Luminal B |
| 93 | 53.60 | 54.20 | 23.13 | Luminal B |
| 94 | 53.99 | 50.26 | 20.41 | Luminal B |
| 95 | 3.76 | 24.18 | 10.20 | Luminal B |
| 96 | 87.97 | 83.37 | 78.75 | Luminal B |
| 97 | 57.53 | 52.67 | 33.35 | Luminal B |
| 98 | 52.90 | 47.66 | 3.69 | Luminal B |
| 99 | 21.99 | 5.90 | 1.04 | Luminal B |
| 100 | 69.16 | 56.05 | 27.46 | Luminal B |
| 101 | 71.49 | 62.97 | 43.73 | Luminal B |
| 102 | 40.26 | 38.00 | 19.17 | TNBC |
| 103 | 21.98 | 56.71 | 29.25 | TNBC |
| 104 | 69.85 | 46.64 | 2.07 | TNBC |
| 105 | 64.01 | 44.33 | 2.38 | TNBC |
| 106 | 61.59 | 12.51 | 14.14 | TNBC |
| 107 | 25.23 | 29.12 | 16.86 | TNBC |
| 108 | 40.33 | 13.25 | 18.11 | TNBC |
| 109 | 66.49 | 35.87 | 2.27 | TNBC |

(continued)

| Sample No. | Fraction marker | | | Subtype |
|---|---|---|---|---|
| | CFTR | CCDC181 | SIM1 | |
| 110 | 64.25 | 36.83 | 2.18 | TNBC |
| 111 | 26.04 | 28.60 | 4.63 | TNBC |
| 112 | 48.39 | 9.85 | 4.10 | TNBC |
| 113 | 13.12 | 44.79 | 2.76 | TNBC |
| 114 | 28.25 | 26.27 | 19.23 | TNBC |
| 115 | 10.07 | 12.75 | 8.84 | TNBC |
| 116 | 2.47 | 13.01 | 2.18 | TNBC |
| 117 | 77.03 | 69.01 | 8.36 | TNBC |
| 118 | 22.33 | 33.04 | 19.13 | TNBC |
| 119 | 53.88 | 18.13 | 4.84 | TNBC |
| 120 | 46.38 | 45.66 | 36.77 | TNBC |

[Table 3-4]

| Sample No. | Fraction marker | | | Subtype |
|---|---|---|---|---|
| | CFTR | CCDC181 | SIM1 | |
| 121 | 40.23 | 22.12 | 3.22 | TNBC |
| 122 | 2.95 | 19.35 | 2.33 | TNBC |
| 123 | 7.73 | 43.47 | 10.65 | TNBC |
| 124 | 73.12 | 9.90 | 2.15 | TNBC |
| 125 | 68.06 | 19.54 | 3.08 | TNBC |
| 126 | 40.05 | 40.93 | 26.96 | TNBC |
| 127 | 41.84 | 60.84 | 35.12 | TNBC |
| 128 | 26.82 | 35.24 | 19.12 | TNBC |
| 129 | 70.52 | 20.94 | 1.25 | TNBC |
| 130 | 61.04 | 59.95 | 27.82 | TNBC |
| 131 | 24.44 | 18.14 | 0.87 | TNBC |
| 132 | 30.22 | 44.65 | 25.85 | TNBC |
| 133 | 29.36 | 10.33 | 7.10 | TNBC |
| 134 | 42.37 | 34.06 | 22.24 | TNBC |

In the results above, when each breast cancer subtype having a methylation level of SIM1, CCDC181, CFTR, and a combination thereof (the highest methylation level in the case of a combination) of higher than 20% was determined to be positive, each fraction marker and the ratio of determination of breast cancer (the ratio of breast cancer patients with positive methylation) were listed in the following Table 4.

[Table 4]

| Table 4. Ratio of determination of breast cancer by fraction markers | |
|---|---|
| Fraction marker | Ratio of determination of breast cancer (ratio of breast cancer patients with positive methylation) (%) |
| SIM1 | 47.0 |
| CCDC181 | 78.4 |
| CFTR | 81.3 |
| SIM1+CCDC181 | 81.3 |
| SIM1+CFTR | 84.3 |
| CCDC181+CFTR | 90.3 |
| SIM1+CCDC181+CFTR | 91.0 |

In the case of any of the fraction markers and any of the combinations thereof, it was indicated that the ratio of determination of breast cancer (the ratio of breast cancer patients with positive methylation) was high. In particular, it was indicated that, when two or more kinds of the fraction markers were used in combination, breast cancer was able to be determined with a high probability.

SEQUENCE LISTING FREE TEXT

SEQ ID NO: 1 indicates a nucleotide sequence in a genome region of 1,000 bp upstream and downstream of the CpG site (nucleotide residues at positions 1,000 and 1,001) for measurement of the methylation level of SIM1 gene.

SEQ ID NO: 2 indicates a nucleotide sequence in a genome region of 1,000 bp upstream and downstream of the CpG site (nucleotide residues at positions 1,000 and 1,001) for measurement of the methylation level of CCDC181 gene.

SEQ ID NO: 3 indicates a nucleotide sequence in a genome region of 1,000 bp upstream and downstream of the CpG site (nucleotide residues at positions 1,001 and 1,002) for measurement of the methylation level of CFTR gene.

SEQ ID NO: 4 to SEQ ID NO: 6 respectively indicate nucleotide sequences of a forward primer, a reverse primer, and a sequencing primer for measurement of the methylation level of the CpG site of SIM1 gene.

SEQ ID NO: 7 to SEQ ID NO: 9 respectively indicate nucleotide sequences of a forward primer, a reverse primer, and a sequencing primer for measurement of the methylation level of the CpG site of CCDC181 gene.

SEQ ID NO: 10 to SEQ ID NO: 12 respectively indicate nucleotide sequences of a forward primer, a reverse primer, and a sequencing primer for measurement of the methylation level of the CpG site of CFTR gene.

## Claims

1. A method for estimating a breast cancer cell existence ratio, the method comprising:

   (1) analyzing a methylation level of

      (a) a cytosine residue in a CG portion consisting of nucleotides at positions 1,000 to 1,001 in a nucleotide sequence of SEQ ID NO: 1,
      (b) a cytosine residue in a CG portion consisting of nucleotides at positions 1,000 to 1,001 in a nucleotide sequence of SEQ ID NO: 2,
      (c) a cytosine residue in a CG portion consisting of nucleotides at positions 1,001 to 1,002 in a nucleotide sequence of SEQ ID NO: 3, or
      (d) a combination of the cytosine residues, in a sample derived from a human subject; and

   (2) estimating the breast cancer cell existence ratio in the sample derived from the human subject, based on the methylation level analyzed at (1).

2. The method according to claim 1, wherein the analyzing is carried out using one or two or more means selected from the group consisting of bisulfite, one or more primers, one or more nucleic acid probes, a restriction enzyme, an anti-methylated cytosine antibody, and a nanopore.

3. The method according to claim 1 or 2, wherein the analyzing is carried out by a bisulfite sequencing method, a bisulfite pyrosequencing method, a methylation specific polymerase chain reaction (PCR) method, a restriction enzyme landmark genome scanning (RLGS) method, a single nucleotide primer extension (SNuPE) method, a CpG island microarray method, a MethyLight method, a COBRA method, a mass spectroscopy (mass array) method, use of a methylation specific restriction enzyme, a high resolution melting (HRM) analysis method, a nanopore analysis method, an ICON probe method, a methylation specific MLPA method, or an immunoassay.

**4.** A method for predicting an effect of a medicinal therapy on breast cancer, the method comprising:

(1) measuring a value of a breast cancer marker in a sample derived from a human subject;
(2) analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues, in the sample derived from the human subject;

(3) correcting the value of the breast cancer marker measured at (1) by the methylation level analyzed at (2) to calculate a correction value of the breast cancer marker; and
(4) predicting the effect of the medicinal therapy on breast cancer, based on the correction value of the breast cancer marker corrected at (3).

**5.** A method for determining breast cancer, the method comprising:

(1) analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues, in a sample derived from a human subject; and

(2) estimating a possibility of developing breast cancer, based on the methylation level analyzed at (1).

**6.** A reagent for estimating a breast cancer cell existence ratio, the reagent comprising a means for analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues.

**7.** A reagent for determining breast cancer, the reagent comprising a means for analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues.

**8.** A kit, comprising:

(1) a means for analyzing a methylation level of

(a) a cytosine residue in a CG portion consisting of nucleotides at positions 1,000 to 1,001 in a nucleotide sequence of SEQ ID NO: 1,
(b) a cytosine residue in a CG portion consisting of nucleotides at positions 1,000 to 1,001 in a nucleotide sequence of SEQ ID NO: 2,
(c) a cytosine residue in a CG portion consisting of nucleotides at positions 1,001 to 1,002 in a nucleotide sequence of SEQ ID NO: 3, or
(d) a combination of the cytosine residues; and

(2) a means for measuring a breast cancer marker.

# FIG. 1

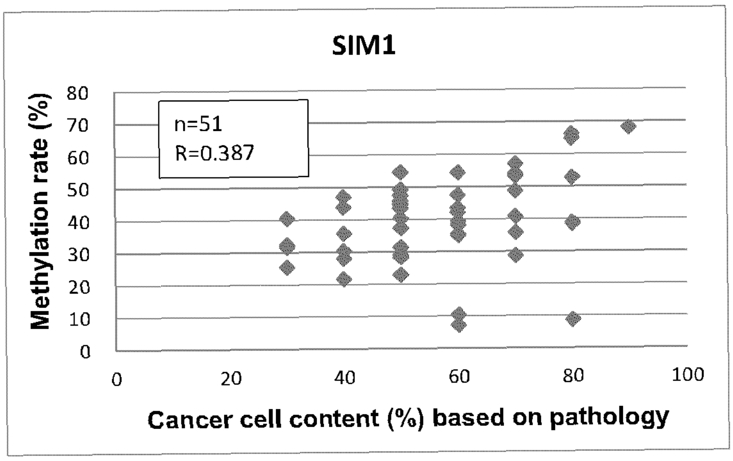
SIM1
n=51
R=0.387
Methylation rate (%)
Cancer cell content (%) based on pathology
0 10 20 30 40 50 60 70 80
0 20 40 60 80 100

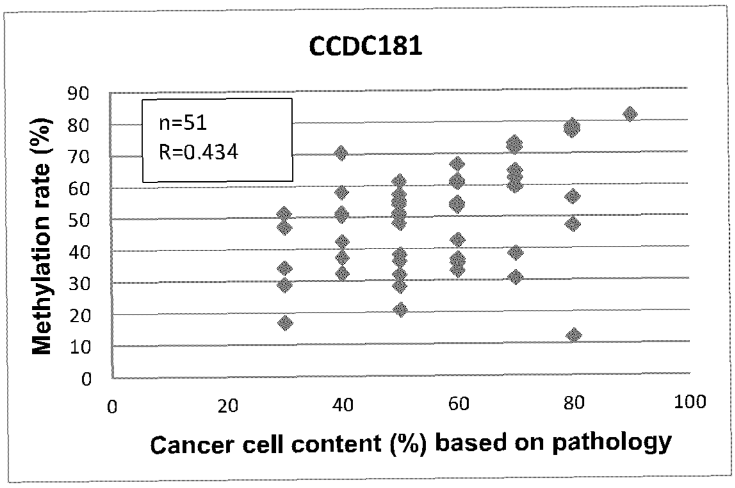
CCDC181
n=51
R=0.434
Methylation rate (%)
Cancer cell content (%) based on pathology
0 10 20 30 40 50 60 70 80 90
0 20 40 60 80 100

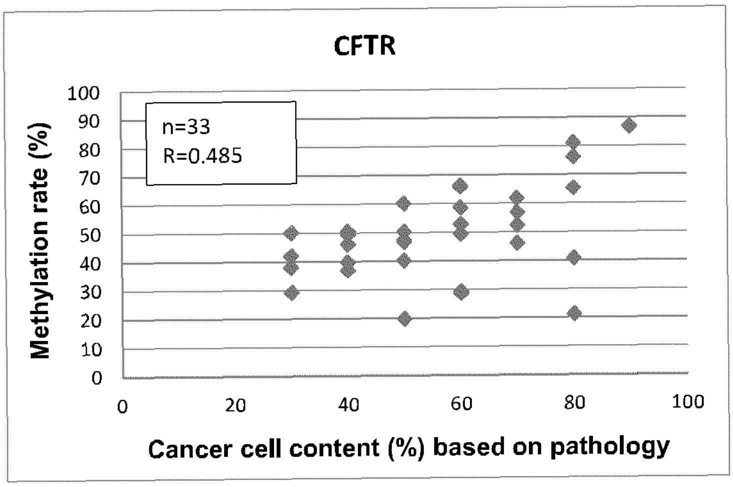
CFTR
n=33
R=0.485
Methylation rate (%)
Cancer cell content (%) based on pathology
0 10 20 30 40 50 60 70 80 90 100
0 20 40 60 80 100

# FIG. 2

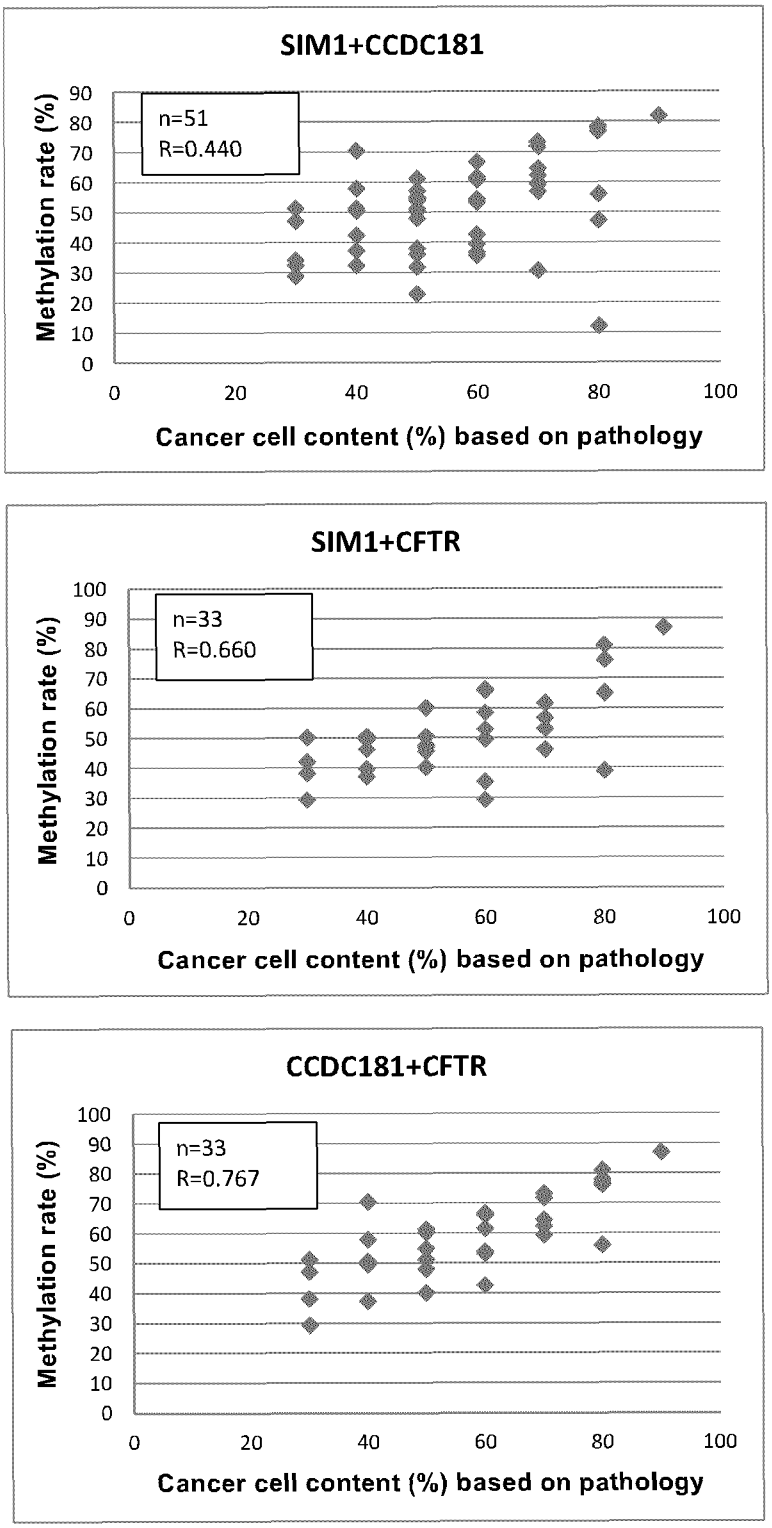
SIM1+CCDC181
n=51
R=0.440
Methylation rate (%)
Cancer cell content (%) based on pathology
SIM1+CFTR
n=33
R=0.660
Methylation rate (%)
Cancer cell content (%) based on pathology
CCDC181+CFTR
n=33
R=0.767
Methylation rate (%)
Cancer cell content (%) based on pathology

FIG. 3

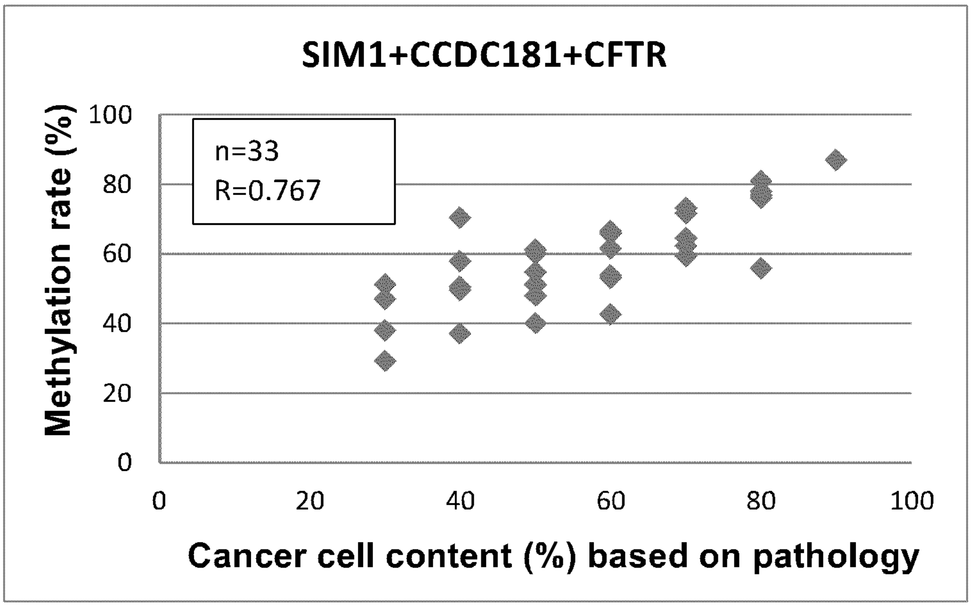
SIM1+CCDC181+CFTR
n=33
R=0.767
Methylation rate (%)
100
80
60
40
20
0
0
20
40
60
80
100
Cancer cell content (%) based on pathology

FIG. 4

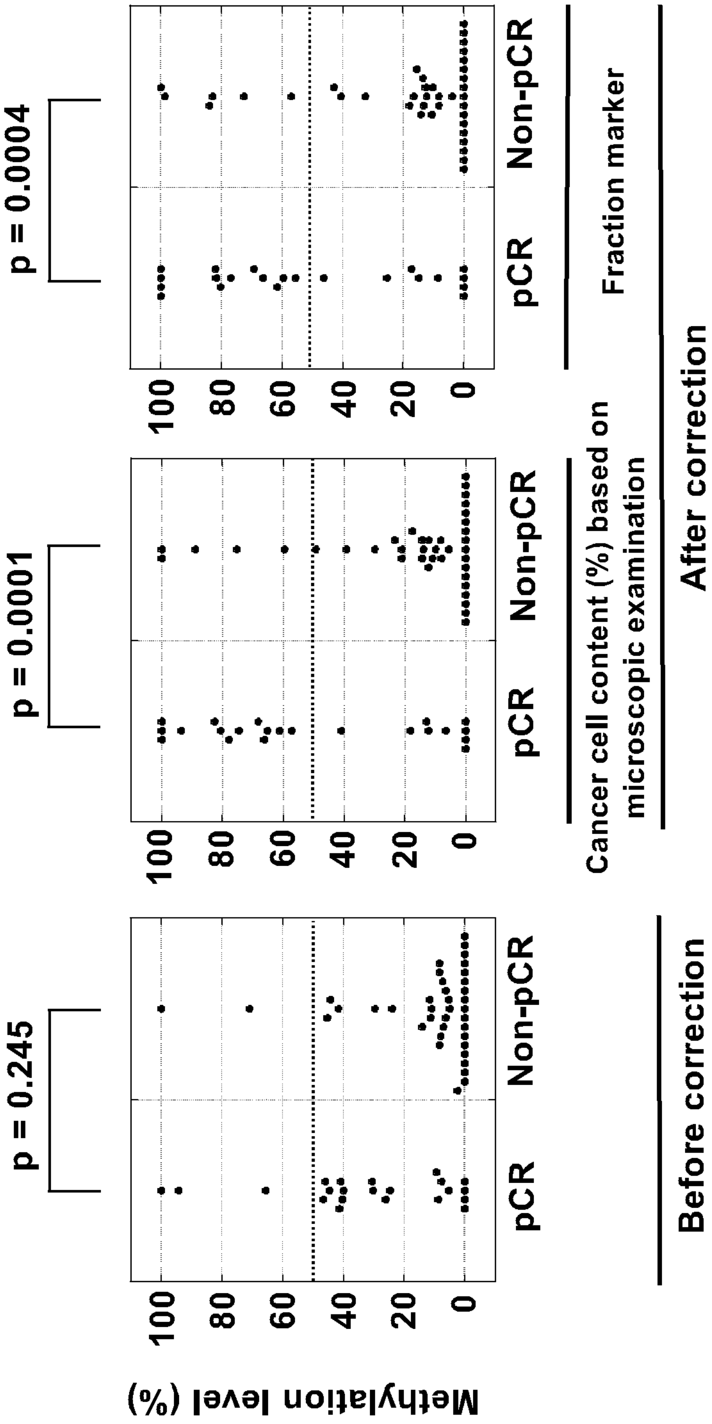
Methylation level (%)
100
80
60
40
20
0
pCR
Non-pCR
p = 0.245
p = 0.0001
p = 0.0004
Before correction
Cancer cell content (%) based on microscopic examination
Fraction marker
After correction

**INTERNATIONAL SEARCH REPORT**

International application No.
PCT/JP2019/035774

A. CLASSIFICATION OF SUBJECT MATTER
Int.Cl. C12Q1/6869(2018.01)i, A61P15/00(2006.01)i, A61P35/00(2006.01)i, G01N33/50(2006.01)i, G01N33/53(2006.01)i, G01N33/574(2006.01)i, A61K45/00(2006.01)n, C12N15/12(2006.01)n
According to International Patent Classification (IPC) or to both national classification and IPC

B. FIELDS SEARCHED
Minimum documentation searched (classification system followed by classification symbols)
Int.Cl. C12Q1/6869, A61P15/00, A61P35/00, G01N33/50, G01N33/53, G01N33/574, A61K45/00, C12N15/12

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | MIYAMOTO, Kazuaki et al., Identification of 20 genes aberrantly methylated in human breast cancers, Int. J. Cancer, 2005, vol. 116, pp. 407-414 | 1-8 |
| A | DEDEURWAERDER, S., DNA methylation profiling reveals a predominant immune component in breast cancers, EMBO Mol. Med., 2011, vol. 3, pp. 726-741 | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27.09.2019 | 08.10.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/035774

C (Continuation). DOCUMENTS CONSIDERED TO BE RELEVANT

| Category* | Citation of document, with indication, where appropriate, of the relevant passages | Relevant to claim No. |
|---|---|---|
| A | DING, S. et al., Methylation profile of the promoter CpG islands of 14 “drug-resistance” genes in hepatocellular carcinoma, World J Gastroenterol, 2004, vol. 10, no. 23, pp. 3433-3440 | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012090555 A
- JP 2014036672 A
- JP 2010538638 PCT
- JP 2009136501 W
- WO 2015025862 A
- WO 2015025863 A
- WO 2015025864 A
- WO 2016052368 A
- JP 2012230019 A
- JP 2010538638 W


### Non-patent literature cited in the description


- **FUJII S ; YAMASHITA S ; YAMAGUCHI T ; TAKAHASHI M ; HOZUMI Y ; USHIJIMA T et al.** Pathological complete response of HER2-positive breast cancer to trastuzumab and chemotherapy can be predicted by HSD17B4 methylation. *Oncotarget,* 2017, vol. 8 (12), 19039-48
- **TAKAHASHI T et al.** *PLoS one,* 2013, vol. 8 (12), e82302
- **ZONG L et al.** *Gastric cancer,* 2016, vol. 19 (2), 361-9
- *DNA Research,* 2006, vol. 13, 37-42
- *Anal. Chem.,* 2012, vol. 84, 7533-7538
- **COUSSENS et al.** *Science,* 1985, vol. 230 (4730), 1132-1139
- **YAMASHITA S ; TAKAHASHI S ; MCDONELL N ; WATANABE N ; NIWA T ; HOSOYA K et al.** Methylation silencing of transforming growth factor-beta receptor type II in rat prostate cancers. *Cancer research,* 2008, vol. 68 (7), 2112-21
- **YOSHIDA T ; YAMASHITA S ; TAKAMURA-ENYA T ; NIWA T ; ANDO T ; ENOMOTO S et al.** Alu and Satalpha hypomethylation in Helicobacter pylori-infected gastric mucosae. *International journal of cancer,* 2011, vol. 128 (1), 33-9